# EUROPEAN PATENT APPLICATION

(11) **EP 1 862 178 A1**
(43) Date of publication of application: **05.12.2007**
(21) Application number: 06115024.9
(22) Date of filing: 06.06.2006
(51) Int. Cl.: A61K 39/395, C12P 21/08, C07K 14/47, C07K 16/18, A61P 35/00, G01N 33/574, G01N 33/577

(54) **Use of HLTF antibodies for diagnosis and treatment of cancer**

(30) Priority: 07.12.2005 EP 05447273
(71) Applicant: Université de Mons-Hainaut, 7000 Mons (BE); UNIVERSITE LIBRE DE BRUXELLES, 1050 Brussels (BE)
(72) Inventor: Belayew, Alexandra, 4130 Esneux (BE); Gerbaux, Cécile, 7504 Froidmont (BE); Wiedig, Murielle, 7011 Ghlin (BE); Debauve, Gaël, 6182 Souvret (BE); Ribaucour, Fabrice, 7000 Mons (BE); Toubeau, Gérard, 7400 Genly (BE); Nonclercq, Denis, 7181 Feluy (BE); Leo, Oberdan, 1970 Wezembeek-Oppem (BE); Laurent, Guy, 1410 Waterloo (BE)
(74) Representative: pronovem

(57) **Abstract**

The invention is related to cancer diagnosis and therapy, especially peptides and antibodies that could be used in the diagnosis and/or the treatment of cancer.

SEQUENCE LISTING

<110> Université de Mons Hainaut
Université Libre de Bruxelles

<120> Diagnosis and Treatment of Cancer

<130> BP.UMHH.010C/EP

<160> 10

<170> PatentIn version 3.3

<210> 1
<211> 20
<212> DNA
<213> Artificial Sequence

<220>
<223> Primer

<400> 1
ttgcagactg tccagtatgg 20

<210> 2
<211> 20
<212> DNA
<213> Artificial Sequence

<220>
<223> Primer

<400> 2
actggcatac tatagcttgg 20

<210> 3
<211> 19
<212> DNA
<213> Artificial Sequence

<220>
<223> Primer

<400> 3
gctgttcagt tgtcatctg 19

<210> 4
<211> 20
<212> DNA
<213> Artificial Sequence

<220>
<223> Primer

<400> 4
actggcatac tatagcttgg 20

<210> 5
<211> 20
<212> DNA
<213> Artificial Sequence

<220>
<223> Primer

<400>  5
ttgcagactg tccagtatgg                                                    20

<210>  6
<211>  20
<212>  DNA
<213>  Artificial Sequence

<220>
<223>  Primer

<400>  6
cccgtgtaat agcgtagtcc                                                    20

<210>  7
<211>  149
<212>  PRT
<213>  Artificial Sequence

<220>
<223>  HLTF8F1 Heavy Chain variable region

<400>  7

Met Lys Cys Ser Trp Val Ile Phe Phe Leu Met Ala Val Val Ile Gly
1               5                   10                  15

Ile Asn Ser Glu Val Gln Leu Gln Gln Ser Gly Ala Glu Leu Val Arg
            20                  25                  30

Ser Gly Ala Ser Ile Lys Leu Ser Cys Thr Ala Ser Gly Phe Asn Ile
        35                  40                  45

Lys Asp Tyr Tyr Met His Trp Val Lys Gln Arg Pro Glu Gln Gly Leu
    50                  55                  60

Glu Trp Ile Gly Trp Ile Asp Pro Glu Asn Gly Asp Thr Asp Tyr Ala
65                  70                  75                  80

Pro Lys Phe Gln Gly Lys Ala Ile Met Thr Ala Asp Thr Ser Ser Asn
                85                  90                  95

Thr Ala Tyr Leu His Leu Ser Ser Leu Thr Ser Glu Asp Thr Ala Val
            100                 105                 110

Tyr Tyr Cys Asn Arg Tyr Asp Tyr Ala Met Asp Tyr Trp Gly Gln Gly
        115                 120                 125

Thr Ser Val Thr Val Ser Ser Ala Lys Thr Thr Pro Pro Ser Val Tyr
    130                 135                 140

Pro Leu Ala Pro Val
145

<210>  8

<211>  134
<212>  PRT
<213>  Artificial Sequence

<220>
<223>  HLTF8F1 Light Chain variable region

<400>  8

Met Met Ser Pro Ala Gln Phe Leu Leu Leu Leu Val Leu Trp Ile Arg
1               5                   10                  15

Glu Thr Asn Gly Asp Val Val Met Thr Gln Thr Pro Leu Thr Leu Ser
            20                  25                  30

Val Thr Ile Gly Gln Pro Ala Ser Ile Ser Cys Lys Ser Ser Gln Ser
        35                  40                  45

Leu Leu His Ser Asp Gly Lys Thr Tyr Leu Asn Trp Leu Leu Gln Arg
    50                  55                  60

Pro Gly Gln Ser Pro Lys Arg Leu Ile Tyr Leu Val Ser Lys Leu Asp
65                  70                  75                  80

Ser Gly Val Pro Asp Arg Phe Thr Gly Ser Gly Ser Gly Thr Asp Phe
                85                  90                  95

Thr Leu Lys Ile Ser Arg Val Glu Ala Glu Asp Leu Gly Val Tyr Tyr
            100                 105                 110

Cys Trp Gln Gly Ala His Phe Pro Gln Thr Phe Gly Gly Gly Thr Lys
        115                 120                 125

Leu Glu Ile Arg Arg Ala
    130

<210>  9
<211>  493
<212>  DNA
<213>  Artificial Sequence

<220>
<223>  HLTF8F1 Heavy Chain variable region

<400>  9
atgaacactg ttttctctac agtcactgaa tctcaaggtc cttacaatga aatgcagctg      60

ggtcatcttc ttcctgatgg cagtggttat aggaatcaat tcagaggttc agctgcagca     120

gtctggggca gagcttgtga ggtcaggggc ctcaatcaag ttgtcctgca cagcttctgg     180

cttcaacatt aaagactact atatgcactg ggtgaaacag aggcctgaac agggcctgga     240

gtggattgga tggattgatc ctgagaatgg tgatactgat tatgccccga agttccaggg     300

caaggccatt atgactgcag acacatcctc caacacagcc tacctacatc tcagcagcct     360

gacatctgag gacactgccg tctattactg taataggtac gactatgcta tggactactg     420

gggtcaagga acctcagtca ccgtctcctc agccaaaacg acacccccat ctgtctatcc 480

actggctcct gtg 493

<210> 10
<211> 431
<212> DNA
<213> Artificial Sequence

<220>
<223> HLTF8F1 Light Chain variable region

<400> 10
gatcactctc ctatgttcat tccctcaaaa tgatgagtcc tgcccagttc ctgcttctgt 60

tagtgctctg gattcgggaa accaacggtg atgttgtgat gacccagact ccactcactt 120

tgtcggttac cattggacaa ccagcctcca tctcttgcaa gtcaagtcag agcctcttac 180

atagtgatgg aaagacatat ttgaattggt tgttacagag gccaggccag tctccaaagc 240

gcctaatcta tctggtgtct aaactggact ctggagtccc tgacaggttc actggcagtg 300

gatcagggac agatttcaca ctgaaaatca gcagagtgga ggctgaggat ttgggagttt 360

attattgctg gcaaggtgca cattttcctc agacgttcgg tggaggcacc aagctggaaa 420

tcagacgggc t 431

## Description

### Field of the invention

The present invention is related to cancer diagnosis and therapy, especially peptides and antibodies that could be used in the diagnosis and/or the treatment of cancer.

### Background of the invention and state of the art

HLTF is a protein that displays motifs of the SWI/SNF family of global transcriptional activators and binds to the promoters of various genes. HLTF nucleotides and amino acids sequences are identified in Genbank accession N° NM 003071 and described in WO03/002068.

The international patent application WO03/002068 describes method for the diagnosis and/or the treatment of cancer, especially by measuring the level of HLTF in the tissue of a mammal, preferably said tissue is breast tissue or skin tissue. This patent application also describes a method for inhibiting carcinogenesis of a cell, comprising reducing the amount of HLTF in the cell, wherein the reduction of HLTF inhibits carcinogenesis.

This patent application also describes oligonucleotides which could be used for reducing the amount of HLTF in the cell. This patent application also states that it is possible to use antibodies directed against an HLTF peptide, or protein domain (epitope) to interfere with its activity or induce its degradation.

### Summary of the invention

The present invention is related to a HLTF protein domain which is specifically recognized by monoclonal antibodies. Said HLTF domain starts from amino acid 1 up to amino acid 270 included of the HLTF sequence.

Preferably, said domain starts from amino acid 1 to amino acid 208 included or starts from amino acid 123 to amino acid 270 included of the HLTF sequence.

Preferably, said HLTF domain is recognized by different monoclonal antibodies which are specifically directed against the domain starting from amino acid 1 to amino acid 208 included, or the HLTF domain starting from amino acid 123 to amino acid 270 included.

The present invention is also related to shorter sequences of HLTF comprised in this amino acid domain; preferably, this shorter sequences are epitopes comprised of at least 6 amino acids (shorter sequences to be recognized as an epitope by a (monoclonal) antibody or a hypervariable portion thereof). Preferably, said sequences comprise at least 7, 8, 9, 10, 20, 25, 30, 35, 40, 45, 50, 55, 60, 80, 100, 120, 150 or 200 amino acids of the above described amino acid domain of HLTF.

These HLTF domains are advantageously specific of the HLTF protein and are present in its isoforms. Therefore, it is possible to use these specific binding domains for obtaining a discrimination between HLTF and other amino acid sequences of the SWI/SNF family.

The HLTF polypeptide sequence according to the invention is identified in Genebank accession n° NM003071 and is described in the patent application WO03/002068.

An isoform of HLTF polypeptide is at least 75%, 80%, 90%, 95%, or 99% identical to the amino acid sequence described in WO03/002068. Amino acid comparisons described herein are carried out using the Lasergene software package (DNASTAR, Inc., Madison, WI). The MegAlign module used was the Clustal V method (Higgins et al. (1989) CABIOS 5(2):151-153). The parameters used are gap penalty 10 and gap length penalty 10.

The domain of the HLTF polypeptide above described could be used for the screening of inhibitors (compounds directed against this HLTF domain). Examples of such inhibitors are (monoclonal) antibodies or a hypervariable portion thereof (antigen binding fragments (e. g. Fab, F(ab')2, ...)).

These inhibitors are possibly linked to a carrier or vector molecule, (possibly labeled or being a contrast agent for imaging), or to a solid support.

Examples of inhibitors that are specifically directed against this HLTF domain are the hypervariable portion of an antibody corresponding to the sequence SEQ.ID.N°7 or SEQ.ID.N°8, possibly linked together.

Other examples of inhibitors directed against this domain are full antibodies (possibly comprising the hypervariable portion above described).

Preferably, these antibodies are monoclonal antibodies, possibly obtained from hybridoma cells having the deposit n° LMBP 6387CB or LMBP 6388CB.

A further example of such inhibitor is any peptide or aptamer (or spiegelmer) that specifically binds this domain of HLTF protein (and most of its isoforms, preferably all of its isoforms). This peptide inhibitor can be selected by phage display technology. This inhibitor includes also oligonucleotides such as siRNA, miRNA, ribozymes, ... that could bind to the nucleotide sequence encoding this domain of HLTF protein and avoids its expression.

Another aspect of the present invention is related to an antiserum targeting said HLTF domain, and which is made of the polyclonal or monoclonal antibodies according to the invention.

Another aspect of the present invention is related to these various inhibitors interacting with the HLTF domain for use as a medicament or for use in diagnosis.

A further aspect of the present invention is related to a pharmaceutical composition comprising an adequate pharmaceutical carrier or diluent and at least one of these inhibitors (peptides, oligonucleotides, antibodies or hypervariable portions thereof) that are possibly labeled, or the antiserum above described).

Another aspect of the present invention is related to a method for the treatment of a mammal (including the human) suffering from a neoplasm (tumor), especially a tumor selected from the group consisting of a HPV-containing lesion, a skin, a breast, a kidney , an ovary, a prostate tissue tumor, a tumor derived from neuronal cells or a brain tumor, or presenting a predisposition to develop this tumor; this method comprising the steps of administrating to the mammal a sufficient amount of the pharmaceutical composition according to the invention for reducing or suppressing the symptoms induced by the tumor.

A method of inhibiting HPV-mediated (or HPV-independent) carcinogenesis of a cell is carried out by reducing the amount of HLTF (or isoforms thereof) in a target cell. The target cell is one which is malignant or suspected of becoming so, (e.g. an HPV-infected cell). A reduction in HLTF in a target cell (including an HPV-infected cell), inhibits carcinogenesis. By carcinogenesis is meant progression from a non-tumor cell to a tumor cell. The term also encompasses progression of a non-malignant tumor cell to a malignant tumor and to metastases.

Methods of reducing the amount of HLTF or inhibiting its activity in a cell include the step of contacting the cell with the composition of the invention. Non-malignant tumors, e.g., warts, are also treated as described herein. For example, an antibody or oligonucleotide directed against an HLTF sequence according to the invention is locally administered to a wart (e.g., as a topical cream or directly injected into a lesion) to kill HPV-infected cells or inhibit the growth of HPV-infected cells and to retard the growth of the lesion.

Another aspect of the present invention is related to the use of the pharmaceutical composition according to the invention for the manufacture of a medicament, for the treatment and/or the prevention of a neoplasm (tumor) in a mammal subject, including a human patient.

Another aspect of the present invention concerns the diagnosis based upon the use of the elements of the invention. Therefore, the present invention is also related to a diagnostic kit for detecting a neoplasm (tumor) in a mammal (including a human patient), preferably an early stage tumor in a mammal, more preferably an early stage tumor of skin cancer, breast cancer, kidney cancer or brain cancer.

The diagnostic kit according to the invention comprises the inhibitor(s) above described (antibodies, aptamers, spiegelmers, oligonucleotides) (possibly bound to a solid support) directed against the above mentioned HLTF domain of the invention.

The diagnostic kit according to the invention may possibly comprise the HLTF domain above described.

All these elements present in the kit could be labeled or could be bound to contrast agents for imaging. In the kit, these labels are present upon molecules that may react with the inhibitors of the invention by all types of detection (especially by ELISA detection, RIA detection, etc).

Optionally, the kit contains a means for determining the amount of HLTF in the sample and means for comparing the amount of HLTF in the sample with a standard control value.

The diagnostic kit comprises also a solid support (possibly bound to the inhibitor(s) (antibodies and interacting molecules such as aptamer, Spiegelmer or oligonucleotide of the invention) such as beads, multiwell plates, labels, detection means, contrast agents for imaging, buffers, washing solutions, etc...

The components of the kit are packaged together in a suitable container. The kit includes instructions for using the inhibitors to detect HLTF protein.

For diagnostic purposes, a body tissue (a body sample obtained from biopsy or a body fluid, such as blood, cerebrospinal fluid, serum or urine), is obtained from an individual suspected of having a malignant tumor or at risk of developing a malignant tumor and tested as described above to determine the level of HLTF in the sample. A test value that is at least 10% greater than the normal control value indicates that the sample contains a malignant tumor. Preferably, the test sample value is at least 25%, 50%, 100%, 500%, 1000%, 2000% or greater than a normal control value (or a value derived from a non-malignant tumor).

For prognostic purposes, e.g., to evaluate a response to therapy, HLTF levels are measured over time. For example, a baseline level is taken prior to the start of therapy and then over the course of therapy (and optionally after therapy is concluded). An increase in HLTF values over time indicates a less favorable prognosis (i.e., an increase in malignancy or unfavorable response to therapy), whereas a reduction in HLTF values over time indicate a more favorable prognosis (e.g., a decrease in malignancy or favorable response to therapy).

A last aspect of the present invention is related to a method for diagnosing a neoplasm (tumor, especially a kidney, ovary, prostate or a brain tumor) in a mammal (including a human patient) which comprises the steps of measuring the level of HLTF in the tissue, especially the kidney, ovary, prostate or brain tissue of said mammal by a binding with the inhibitor or interacting molecule (aptamer, Spiegelmer, oligonucleotide, antibodies, hypervariable portion thereof, antiserum or peptide and possibly bound to a solid support) according to the invention and which are able to specifically recognize the HLTF domain above described.

The method for selecting the presence of a malignant tumor or a predisposition to develop said tumor comprises the steps of contacting a sample obtained from said mammal (including a human patient) by a biopsy or from a body fluid, such as blood, cerebrospinal fluid, serum or urine.

An increase in the level of HLTF, or isoforms thereof, in the test tissue compared to the level in a normal control tissue or fluid indicates the presence of a neoplasm in the tissue. The level of HLTF is determined by detecting an HLTF gene product (polypeptide). The tissue is preferably any tissue suspected of containing a malignant tumor or which contains an HPV-containing lesion.

An increase in binding compared to a normal control level of binding indicates the presence of a (malignant) tumor or a predisposition to developing such a tumor.

The diagnostic method has advantages over existing diagnostic methods. For example, the method allows very early detection of malignancy, e.g., prior to overt physical symptoms or histological changes, especially for a kidney, ovary, prostate or brain tumor. Another advantage is that the method is objective, i.e., a determination of malignancy is based on measured values which are compared to standard control values, rather than subjective viewing of histological samples by a individual such as a clinical pathologist.

The diagnostic method according to the invention is also advantageously applied for the detection of residual cancer cells or cancer relapsing after a therapeutic protocol.

The present invention is related to a method of detecting at an early stage, the presence of a tumor, especially a kidney, ovary, prostate or brain tumor in a mammal, (in particular in a human patient) said method comprising the steps of extracting a biological sample from the said mammal, contacting the biological sample with the inhibitor (or interacting molecule according to the invention and above described), so as to be able to form a complex between the inhibitor (or interacting molecule) and the HLTF domain to which it binds, measuring the level of an HLTF gene product (polypeptide) in the sample of the mammal there through, wherein detecting an increased level of HLTF product in the sample compared to the level in normal control samples, indicates the presence of an early stage tumor, especially a kidney, ovary, prostate or brain tumor, in the biological sample.

### Brief description of the drawings

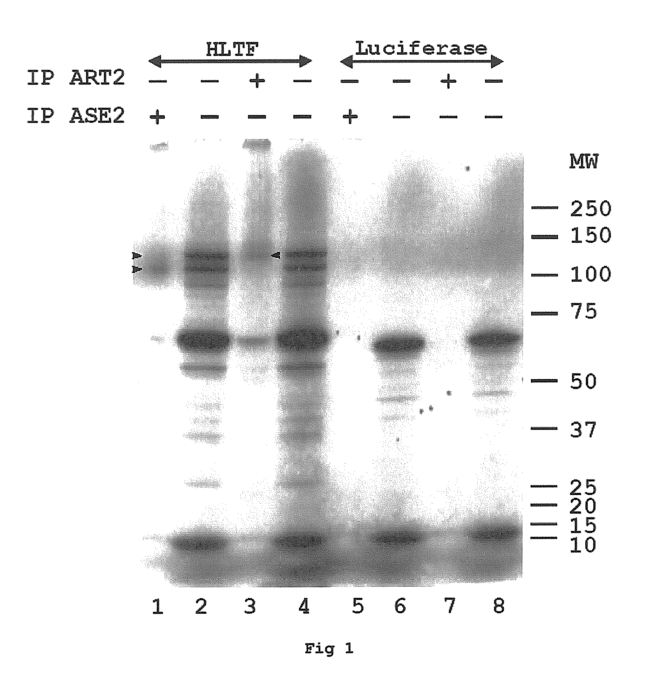

Fig. 1 gives the results of specificity tests of antisera by immunoprecipitation of human HLTF variants expressed by transcription/translation in a rabbit reticulocyte lysate in the presence of [³⁵S] cysteine. In *vitro* transcription/translation was performed in the presence of [³⁵S] cysteine in a TNT rabbit reticulocyte lysate programmed with either *pGEM-4Z-HLTF* encoding both HLTFMet1 and Met123 variants (lanes 1-4) or Luciferase T7 control plasmid (lanes 5-8). The products were resolved by electrophoresis on a 4-12% SDS-PAGE gradient and detected by autoradiography. The samples were either 1,5 µl of total *in vitro* translation products (lanes 2, 4, 6, and 8), or 15 µl of *in vitro* translation products immunoprecipitated either by the ASE2 antiserum directed against HLTFMet123 (lanes 1 and 5) or the ART2 antiserum directed against HLTFMet1 (lanes 3 and 7). The autoradiography was exposed 3 days. The sizes of the molecular weight markers (MW) are given in kDa. Arrowheads indicate the HLTF proteins.

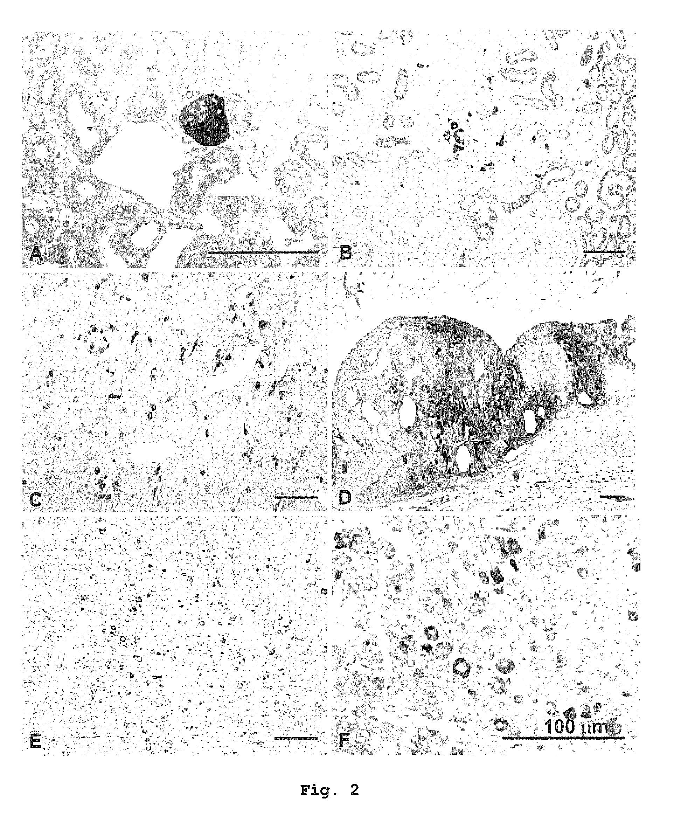

Fig.2 gives the result of HLTF immunostaining experiments in kidney and tumor tissue. A-D, kidneys from DES-treated hamsters; E-F, HKT-1097-derived tumor xenograft. Tissue sections were processed for HLTF immunostaining with antisera directed against either HLTFMet123 (ASE2; A, B, D) or HLTFMet1 (ART2; C, E, F). A, HLTF immunolabeling associated with early signs of neoplastic transformation (tumoral bud; two months of DES exposure). B, C, scattered HLTF-positive cells in large renal tumors (6 months of DES exposure). D, cell populations with HLTF immunoreactivity in a large tumor (11 months of DES exposure). E, F, scattered HLTF-positive cells in subcutaneous tumor xenograft.

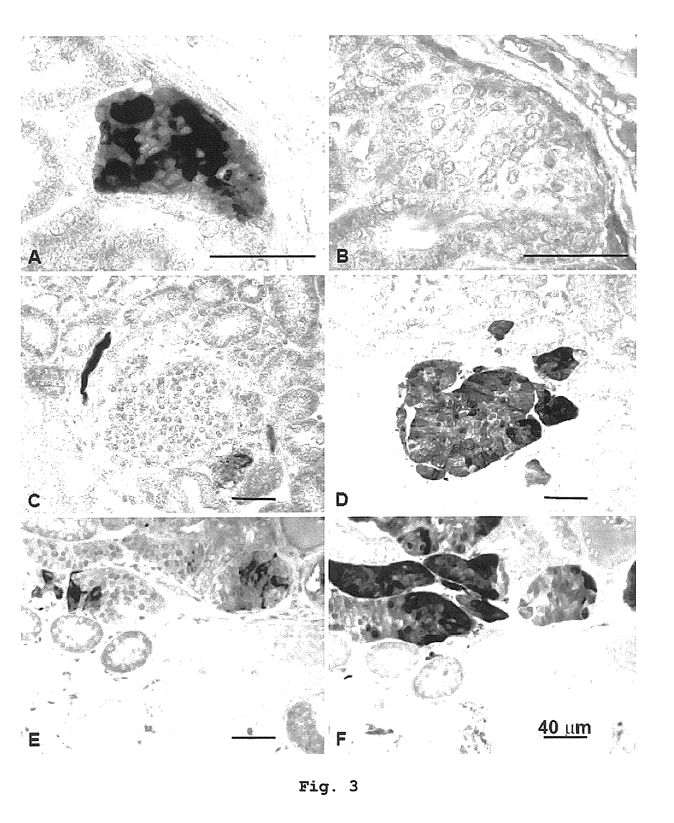

Fig. 3 represents the results of combined immunolocalization experiments of HLTF (A, C, E) and S100 protein (B, D, F) in DES-induced renal tumors. Immunostaining was performed with the ASE2 antiserum directed against HLTFM et123. A-B, C-D, E-F are corresponding fields in consecutive tissue sections. A-B, a cluster of neoplastic cells with HLTF immunoreactivity (A) appears S100 negative (B) (7 months of DES exposure). C-D, a small tumor negative for HLTF (C) exhibits S100 immunoreactivity (D) (7 months of DES exposure). E-F, mixed population of HLTF-positive (E) and S100-positive (F) cells in a medium size tumor (6 months of DES exposure) .

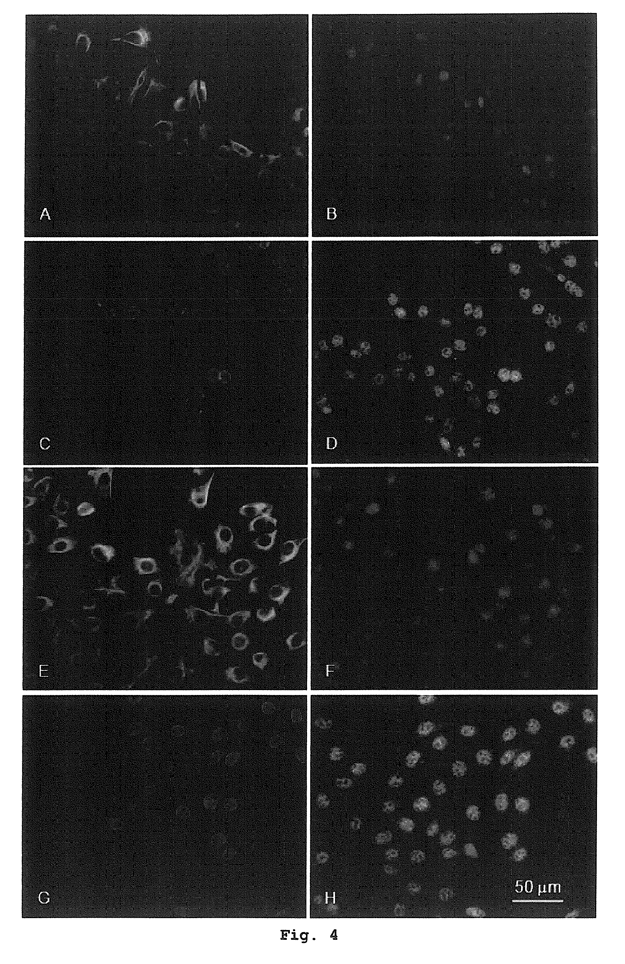

Fig. 4 corresponds to the results of immunofluorescence staining experiments of HLTF in renal tumor-derived HKT-1097 cells. Cultured cells were processed for double immunofluorescence staining as described in Materials and methods. A, E: general cell appearance revealed by vimentin immunostaining (FITC immunolabeling); B, F: virtually all cells in the microscopic fields display HLTF immunoreactivity as shown by application of antisera (Texas Red labeling) directed against either HLTFMet123 (ASE2; B) or HLTFMet1 (ART2; F). C, G: cell nuclei immunolabeled with anti-lamin B serum (Texas Red labeling); D, H: use of ASE2 (D) or ART2 (H) (FITC labeling) reveals the nuclear localization of immunoreactive HLTF variants.

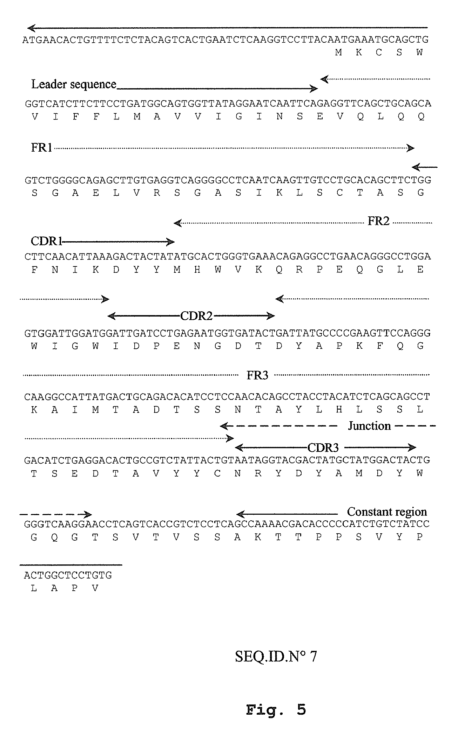

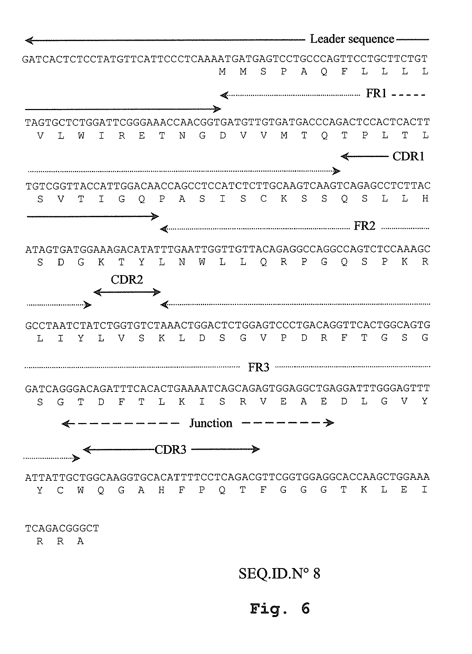

Fig. 5 and 6 represent respectively the heavy chain and light chain variable region of HLTF8F1 monoclonal antibody.

### Detailed description of the invention

An antibody, according to the invention, that specifically binds to HLTF is used. An intact antibody, or any antigen-binding fragment thereof (e.g., Fab or F(ab')₂) can be used. Monoclonal antibodies, which specifically bind to HLTF were generated using standard methods. The antibody specifically detects HLTF in immunoblots and immunoprecipitation assays. The antibody binds a HLTF domain with HLTF amino acids 1 to 208 or 123 to 270.

The term "labeled", with regard to the antibody, encompasses direct labeling of the antibody by coupling (i.e., physically linking) a detectable substance to the antibody, as well as indirect labeling of the antibody by reactivity with another reagent that is directly labeled. Examples of indirect labeling include detection of a primary antibody using a fluorescently labeled secondary antibody. The assays are carried out *in vitro,* ex *vivo,* or *in vivo. In vivo* techniques for detection of HLTF include introducing into a subject a labeled anti-HLTF antibody. For example, the antibody is labeled with a radioactive marker or a contrast agent whose presence and location in a subject can be detected by standard imaging techniques.

In addition to solid tissue biopsies, body fluid such as blood, serum, cerebrospinal fluid and urine is tested. HLTF protein is present in bodily fluids such as blood following necrosis of some cells of a tumor nodule and release of the protein (which is normally present in the nucleus or cytoplasm). This phenomenon occurs at the cancer progression stage at which the tumor is too large for its central core cells to get proper food and oxygenation. As the tumor grows, it recruits blood vessels (angiogenesis) to supply the large food and oxygen amounts needed for accelerated growth. At the time new blood vessels contact the necrotic cells, HLTF is release into the blood stream. HLTF in the blood and other bodily fluids is assayed using known methods, e.g., ELISA. In another example, HLTF is measured in urine for diagnosis of cancers such as prostate or bladder cancer. Antibodies against HLTF that patients might have generated could also be detected by ELISA using as antigen an HLTF protein fragment expressed in heterologous cells, such as E. coli.

The control sample is derived from a bodily tissue of an individual who is known not to have a cancer (e.g., a malignant tumor) based on earlier efforts to diagnose such pathologies. Alternatively, the control amount is an average of values from a plurality of non-cancerous individuals. To distinguish between benign and malignant tumors, the control amount is an amount derived from a benign tumor or a plurality of benign tumors.

### Therapeutic compositions and methods

To prevent or treat a disease or condition associated with an HPV infection (e.g., a wart or an HPV-associated malignant tumor) or an aberrant HLTF expression or activity (e.g., breast cancer), a compound, such as the peptide, the antibody or the antiserum according to the invention which modulates either HLTF expression, HLTF protein activity or degradation, is administered. A suspicious lesion or growth is contacted directly with the compound, e.g., by injecting the compound directly into the site of the lesion, or by administrating the compound systemically.

Subjects to be treated are suffering from or at risk for a disease which is caused or contributed by aberrant HLTF expression or activity. Such individuals are identified by any or a combination of diagnostic or prognostic assays described herein. An HPV-infected individual (without an apparent tumor or HPV-associated lesion) is also at risk of developing an HPV-associated malignant tumor. HLTF-inhibitory compounds are optionally administered prior to the manifestation of symptoms characteristic of the HLTF aberrancy to prevent progression to malignancy or after detection of malignancy to delay its progression.

Parenteral administration, such as intravenous, subcutaneous, intramuscular, and intraperitoneal delivery routes, are used to deliver HLTF-inhibitory peptides, antibodies or antiserums compounds, aptamers, spiegelmer, oligonucleotide or compositions. Direct infusion into the tumor site, e.g., a skin lesion, is carried out for local delivery of the therapeutic agent.

Dosages for any one patient depends upon many factors, including the patient's size, body surface area, age, sex, time and route of administration, general health, and other drugs being administered concurrently.

### Production of HLTF-specific antibodies

Anti-HLTF antibodies are obtained by techniques well known in the art. Such antibodies are polyclonal or monoclonal. Polyclonal antibodies which bind to HLTF were obtained using standard methods, e.g., by the methods described in Ghose et al. (1983), Methods in Enzymology, 93:326-327. HLTF peptides were coupled to KLH and used as immunogen to stimulate the production of polyclonal antibodies in the sera of rabbits (e.g., using the method described by Ribaucourt et al.) Antigenic polypeptides for production of both polyclonal and monoclonal antibodies useful as immunogen include HLTF domains, such as amino acids 1-208 or amino acids 123-270 (as shown in fig. 5).

Antibody-producing hybridomas are made by using standard methods from mice immunized with HLTF domains such as amino acids 1-208 or amino acids 123-270 expressed in E. coli. To identify those hybridomas producing antibodies that are highly specific for an HLTF polypeptide, hybridomas are screened using the same immunogen polypeptide used to immunize or a full-length HLTF protein. A preferred antibody has a binding affinity of at least about 10⁸ liters/mole and more preferably, an affinity of at least about 10⁹ liters/mole.

Monoclonal antibodies are humanized by methods known in the art, e.g, MAbs with a desired binding specificity can be commercially humanized (Scotgene, Scotland; Oxford Molecular, Palo Alto, CA; Dyax, Cambridge, MA).

HLTF-specific monoclonal antibodies are expressed in target cells intracellularly as follows.

Following identification of a hybridoma producing a suitable monoclonal antibody, DNA encoding the antibody is cloned. For this purpose, messenger RNA's are extracted and their 5' region is amplified by 5' RACE (rapid amplification of 5' end) using known methods with the following steps: (i) an upstream primer is ligated to the mRNA 5' end; (ii) a DNA complementary to the messenger RNA is synthesized by reverse transcription starting from a downstream primer specific for the conserved region of the immunoglobulin class to which the monoclonal antibody belongs (Hawkins et al. (1994), Blood 83:3279-3288 and Sahota et al. (1997), Blood 89:219-226 and Zhu et al. (1998) Immunol. 93:162-170); (iii) an amplification is performed by the polymerase chain reaction with the upstream and downstream primers; (iv) the resulting fragment is cloned in a plasmid and its sequence is determined. This method is applied for the messenger RNA encoding the antibody heavy chain and for the messenger RNA encoding the antibody light chain. DNA encoding a single chain HLTF-specific antibody in which heavy and light chain variable domains are separated by a flexible linker peptide is cloned into an expression vector using known methods (e.g., Marasco et al. (1993), Proc. Natl. Acad. Sci. USA 90:7889-7893 and Marasco et al. (1997), Gene Therapy 4:11-15). Such constructs are introduced into target cells, e.g., using standard gene delivery techniques for intracellular production of the antibodies. Intracellular antibodies are used to inhibit HLTF activity e.g. transactivation of target genes such as hTERT promoter by endogenous cellular HLTF or its isoforms.

### Early expression of HLTF in an experimental model of estrogen-induced renal carcinogenesis:

### Materials and methods

### Cell culture

The major characteristics of the HKT-1097 cell line (ECAAC n° 98061003, DSMZ n° AAC 445) were described in previous publications (Brohee,R et al. (2000) In Vitro Cell Dev.Biol.Anim, 36, 640-649; Laurent,G et al. (1999) In Vitro Cell Dev.Biol.Anim, 35, 339-345). Cell cultures were maintained at 37°C in a cell incubator with humid atmosphere at 5% CO₂. Cells were propagated in T75-flasks (Orange Scientific, Braine-l'Alleud, Belgium) containing Dulbecco's Modified Essential Medium (DMEM, BioWhittaker Europe, Verviers, Belgium) supplemented with Phenol Red, 10 % fetal bovine serum (FBS, HyClone, Logan, Utah), 25 mM N-2-hydroxyethylpiperazine-N'-2-ethanesulfonic acid (HEPES), 2 mM L-glutamine, 100 U/ml penicillin G, 100 µg/ml streptomycin, and 0.25 µg/ml amphotericin B (DMEM-FBS) (supplements from BioWhittaker or Gibco.-Invitrogen, Merelbeke, Belgium). Cell cultures were passed once a week (split ratio 1:300) and were fed fresh medium every 2-3 days. For routine subculture and cell plating in preparation of immunofluorescence studies, cells were dislodged from the vessel bottom by treatment with trypsin-EDTA solution, suspended in culture medium by vigorous pipetting and counted in a model Z1 Coulter counter.

### Animals and treatment

It should be noted that in the present invention, it is meant by "early expression of HLTF product in an experimental animal model (hamster) of estrogen-induced renal carcinogenesis" an expression of HLTF already detected in small tumour buds consisting of only a few dozen cells.

Male Syrian hamsters *(Mesocricetus auratus)* weighting 70-80 g (3-4 month old) were used throughout the study. The animals were bred and maintained at the UMH animal facility accredited (n°LA 1500021) by the Belgian Ministry of Middle Class and Agriculture, and the experiments performed in compliance with its guidelines. Five groups of at least 4 animals received chronic treatment with DES following a protocol detailed previously (Nonclercq,D. et al. (1998) Eur.J.Morphol., 36, 83-96). Implants filled with 25 mg of DES (Sigma; S^{t} Louis, MO) were inserted in the subcutaneous tissue of the shoulder area of anesthetized animals and renewed every 2.5 months to maintain a constant blood level of DES (approx. 13ng/ml). Hamsters (at least 4 animals per time point) were terminated 1, 2, 4, 5, 6, 7, 9, 10 and 11 months after the first implantation. A group of sham-operated animals (n=4) and a group of untreated animals (n=4) of various ages (7-9 months) were included as controls. *Antisera*

Two putative immunogenic peptides corresponding to the individual HLTF variants (see Result section) were defined by accessibility prediction programs (Garnier software from the EMBOSS software package; (Garnier, J. et al. (1978) J Mol Biol., 120, 97-120), synthesized at Eurogentec (Seraing, Belgium) and conjugated to a carrier protein (keyhole limpet hemocyanin). Two rabbits were challenged by three intradermal injections of each conjugate at two-week intervals, followed by a booster inoculation one month later. The animals were bled 10 days after the last immunization. The rabbits were housed in the IBMM (Gosselies, Belgium) animal facility according to standard protocols and in agreement with the Animal Care and Use Committee of the Free University of Brussels (n°LA 1500474).

### Transcription/translation Assays

*In vitro* transcription/translation assays were carried out in a TNT rabbit reticulocyte lysate (Promega Corporation, Madison, WI, USA) in the presence of T7 RNA polymerase and [³⁵S] cysteine (1,000 Ci/mM; Amersham Biosciences, Roosendaal, The Netherlands). The cDNA template used *(pGEM-4Z-HLTF)* has been previously described and encodes both HLTF variants (Ding et *al.,* 1996). A luciferase cDNA template was substituted for the HLTF cDNA as a control. The reaction mixtures were boiled in sample buffer (2% SDS, 62.5 mM Tris-HCl pH 6.8, 5% β-mercaptoethanol, 20% glycerol, 0.5% bromophenol blue, 18 mM DTE) and resolved by electrophoresis on 4-12% polyacrylamide gels (SDS-PAGE). The gels were treated with Amplify (Amersham Biosciences), and dried for autoradiography on a Kodak BioMax MR film (Amersham Biosciences).

### Immunoprecipitations

For immunoprecipitations, 15 µl [³⁵S]-labelled in *vitro* transcription/translation products were incubated 2 hours at 4°C with 10 µl rabbit antiserum in a 1 ml final volume of 150 mM NaCl, 10 mM Tris, 1 mM EDTA, 0.5 mM EGTA, 1% Triton X-100, 0.5 mM orthovanadate, 0.5 mM PMSF and protease inhibitor cocktail (Roche Diagnostics Be, Vilvoorde, Belgium). This was followed by a further one-hour incubation with protein A-Sepharose (128 mg/ml; Amersham Biosciences). After centrifugation, the pellets were washed 3 times in the immunoprecipitation buffer, and suspended in sample buffer for analysis by SDS-PAGE (4-12% gradient) followed by autoradiography as stated above.

### Hamster HLTF cDNA sequences

PolyA(+) RNA was extracted with the Micro-FastTrack mRNA isolation kit (Invitrogen) from 5x10⁶ HKT-1097 cells. Reverse transcription was performed with random primers and the MMLV reverse transcriptase (Promega) according to the protocol provided by the manufacturer. The primers used for PCR were 5' TTGCAGACTGTCCAGTATGG 3' (forward, SEQ.ID.NO.1) and 5' ACTGGCATACTATAGCTTGG 3' (reverse, SEQ.ID.NO.2); amplification was done with *Pfu* DNA polymerase (Promega) in a Mastercycler gradient (Eppendorf) set for 35 cycles (40 sec at 95°C, 40 sec at 50 °C, 55 sec at 74°C). Protruding A's were added to the PCR product was *Taq* DNA polymerase 15 min at 72°C and it was ligated into the *pCR4-TOPO* vector (Invitrogen). Plasmid DNA was extracted from transformed *E. coli* colonies with the Wizard Plus SV Minipreps DNA Purification System (Promega). Sequences were determined by the dideoxynucleotide chain termination method with CEQ DTCS Quick Start Kit and the CEQ2000 sequencer (Beckman-Coulter). Sequences were analyzed on the Belgian EMBL node (http://ben.vub.ac.be) with the EMBOSS software package.

Rapid amplification of cDNA 5' end was performed on 250 ng polyA(+) RNA with the FirstChoice RLM-RACE kit (Ambion) according to the manufacturer's protocols. The specific *HLTF* primers used were the following: 5' GCTGTTCAGTTGTCATCTG 3' (outer, SEQ.ID.NO.3), 5' ACTGGCATACTATAGCTTGG 3' (inner, SEQ.ID.NO.4). The products were resolved by electrophoresis on a 1% agarose gel, the larger fragment was extracted from the gel with the Qiaquick Gel extraction kit (Qiagen), cloned and sequenced as detailed above.

### Hamster genomic sequences

Genomic DNA was extracted from hamster liver with the Scil DNA Tissue Kit in the KingFisher (ThermoLabs System) apparatus. 700 ng DNA were amplified by *Pfu* DNA polymerase with primers mapping to exon 2: 5' TTGCAGACTGTCCAGTATGG 3' (forward, SEQ.ID.NO.5) and 5' CCCGTGTAATAGCGTAGTCC 3' (reverse, SEQ.ID.NO.6). A denaturation step of 3 min at 95°C was followed by 35 PCR cycles of 30 sec at 94°C, 60 sec at 56°C, 60 sec at 72°C. The resulting 166-bp fragment was cloned and sequenced as detailed above.

### Morphological procedures

### Semiquantitative assessment of DES-induced renal tumors

Neoplasms in hamster kidneys were analyzed as described previously (Toubeau,G. et al. (2001) Histochem.Cell Biol., 115, 429-438) and graded according to size and gross morphology. Briefly, three stages were defined on the basis of the following criteria: 1) Tumoral buds: well-defined, small clusters of tumor cells surrounded by normal kidney tissue (5-200 cells); 2) Medium-sized tumors: more important clusters of tumor cells infiltrating normal kidney tissue (i.e. diameter of 150 - 600 µm); 3) Large invasive tumors of large diameter extensively infiltrating the kidney (>300.000 µm² and up to 80 mm²). These stages are illustrated in Figures 3 and 4.

All reagents for immunohistochemistry (primary antibodies, conjugated antibodies or F(ab)2 fragments, ABC kit) came from Dakopatts (Glostrup, Denmark) except where stated otherwise.

Immediately after sacrifice, kidney samples were fixed by immersion in alcoholic Bouin mixture (Duboscq-Brazil fluid) for at least 48 h and embedded in paraffin according to standard procedures. Sections of 4-5 µm thickness were cut serially with a Reichert Autocut 2040 microtome and mounted on silane-coated glass slides.

Tissue sections were immunostained following a slightly modified version of the streptavidinbiotin immunoperoxidase method (ABC method). The primary antibodies, anti-HLTFMet1 (ART2) and anti-HLTFMet123 (ASE2), were tested in preliminary studies to determine the optimal dilutions, 1/350 and 1/300 respectively. The sensitivity of the method was increased by microwave pre-treatment of dewaxed sections in 0.01 M citrate buffer (pH 6.0) for 2 x 5min at a power of 900 W. After microwave treatment, the sections were incubated in 0.4% hydrogen peroxide for 5 min and rinsed in PBS. Thereafter, the sections were successively exposed to avidin (0.1mg/ml in PBS) and to biotin (0.1mg/ml in PBS) for 20 min to block the reactivity for endogenous biotin. After rinsing in PBS, the sections were incubated in 5% normal goat serum in PBS (NGS-PBS) for 20 min and incubated sequentially at room temperature in the following solutions: (1) primary antiserum for 1 hour; (2) biotinylated goat anti-rabbit IgG (diluted 1:50) for 20 min, and (3) ABC complexes for 20 min. Bound peroxidase activity was visualized by incubation with 0.02% 3, 3'-diaminobenzidine - 0.01% H₂O₂ in PBS. The sections were finally counterstained with PAS, hemalun and luxol fast blue and mounted in a permanent medium.

The simultaneous detection of HLTF proteins and neurofilament proteins by double-label immunofluorescence was performed on serial sections obtained from animals sacrificed after 7 months of DES exposure. The slides were pretreated as described above for the ABC method and then incubated for 1h at room temperature with a mouse monoclonal antibody against neurofilament protein mixed with anti-HLTFMet1 (ART2) or anti-HLTFMet123 (ASE2) rabbit sera to ensure optimal dilutions. In the next step, the sections were incubated for 30 min at room temperature with a mixture containing biotinylated swine anti-rabbit IgG antibody and FITC-conjugated goat anti-mouse IgG antibody both at a final dilution of 1:50. Finally, the slides were incubated 30 min at room temperature with Texas red-conjugated streptavidin (Pierce, Rockford, ILL, USA) at 1:100 dilution. The labeled sections were mounted in Vectashield mounting medium (Vector Laboratories, Inc., Burlingame, CA). Immunostained sections were examined on a Leitz Orthoplan fluorescence microscope (Ploem system) and the positive fields recorded with a digital camera (Leica DC 300F).

### Immunofluorescence staining of cultured cells

For cell immunostaining, twelve-well plates containing sterile round glass coverslips (15-mm diameter) were seeded at a density of 5 x 10³ cells / mm². After 3 days of growth, cell cultures were rinsed with Dulbecco's phosphate buffered saline (DPBS) and fixed at 4°C in 4% paraformaldehyde in DPBS. After 15 min. the fixative was replaced by DPBS where cells were kept at 4°C until immunostaining. For the simultaneous demonstration of vimentin and HLTF, the cells were incubated with a mixture of mouse monoclonal anti-porcine vimentin and rabbit polyclonal anti-HLTF antibodies (ASE2 or ART2). This was followed by exposure to a mixture of fluorescein isothiocyanate (FITC)-conjugated goat anti-mouse immunoglobulins antibodies (F(ab)2 fragments) and biotinylated swine anti-rabbit immunoglobulins antibodies. HLTF-containing immunocomplexes were finally demonstrated by incubating cell preparations with Texas Red-labeled streptavidin. For the simultaneous demonstration of lamin and HLTF, cell preparations were first exposed to anti-HLTF antibodies (ASE2 or ART2), followed by FITC-conjugated swine anti-rabbit immunoglobulins antibodies (Vector Laboratories, Burlingame, CA). The next step consisted in an incubation in presence of rabbit immunoglobulins in order to saturate free binding sites of anti-rabbit immunoglobulins antibodies. Thereafter, cells were exposed to goat polyclonal anti-lamin B antibodies (Santa Cruz Biotechnology, Santa Cruz, CA), followed by biotinylated rabbit anti-goat immunoglobulins antibodies. Finally, lamin-containing immunocomplexes were demonstrated by using Texas-Red-labeled streptavidin as described above. The preparations were mounted and observed as described above for double immunostaining.

Controls for the specificity of immunolabeling included the omission of the primary antibody or the substitution of non-immune sera for the primary antibodies. The specificity of anti-HLTF immunostainings was also checked with primary antibodies previously incubated with the corresponding synthetic peptide used as antigen. In each case these controls were negative.

### Production of hybridoma producing HLTF 8 Fl and HLTF5A8 antibodies:

The HLTF cDNA fragment encoding amino acid 123-270 (NH² terminal domain of HLTFMet123) was cloned into pCRT7/NT-TOPO (Invitrogen, CARLSBAD.CA) downstream of a cDNA fragment encoding His-tag under control of a T7 promoter. The plasmid was introduced in BL21 Star™ (DE3) pLysS (Invitrogen). Upon induction by IPTG addition to the culture medium, bacteria produced high levels of the His-tagged protein in inclusion bodies. The protein was solubilized in 6M urea and purified on His-bind Resin (Novagen). This protein was used as antigen for hybridoma production.

Three Balb/c mice were injected intraperitaneously (ip) with 6µg of antigen after emulsification with Freund's Complete Adjuvant. Mice were given one first booster injection after 8 weeks with 10 µg of the antigen emulsified in Freund's Incomplete Adjuvant and another one after 20 weeks with 10 µg of the antigen emulsified in Freund's Complete Adjuvant. After the third immunization, sera from individual animals were screened for the presence of antibodies directed to the antigen in a standard solid-phase ELISA. The mouse whose serum exhibited the best response in ELISA was chosen. A final intravenously (iv) injection was given with 10 µg of the antigen without adjuvant at 3 days prior to the cell fusion.

Mouse spleen cells were fused with the SP2/0 myeloma cells and B cell hybridomas were propagated according to standard methods. Culture media of the hybridomas were screened for binding to the antigen in a standard solid-phase ELISA. Positive hybridomas were cloned 3 times by limiting dilution until cell lines were clonal and stable antibody producers.

One hybridoma (HLTF8F1) was positive for all screening criteria, including ELISA Western Blot, immunoprecipitation, immunofluorescence and was specifically directed against HLTFMet123, not reacting with HLTFMet1 in non denaturing conditions. Another hybridoma (HLTF5A8) was also positive for all our screening criteria and was specifically directed against HLTFMet123 and HLTFMet1. The cloned cell line HLTF8F1 was mass produced in ascite fluids and purified by affinity chromatography on protein-G-Sepharose (Division Immunologie Animale, C.E.R., Marloie, Belgique).

### Characterization of the antisera

Two human HLTF variants are expressed from the same open reading frame and only differ by the translation start site (Met1 or Met123) (Ding,H. et al. (1996) DNA Cell Biol., 15, 429-442). In order to raise an antiserum (ART2) specific for the HLTFMet1 variant, a putative immunogenic peptide was chosen i.e. residues 42-56 in the amino-terminal sequence missing in the shorter variant. The HLTFMet123 sequence being entirely included in the longer variant, it was supposed that its amino-terminal region might differ in terms of conformation from the corresponding sequence in HLTFMet1. Structure predictions indicated that a set of four putative alpha helices were disrupted by the NH2-truncation in the shorter variant. With the aim to prepare an antiserum (ASE2) specific for HLTFMet123, a putative immunogenic peptide located from residues 152 to 166 was thus selected, surmising that it might be accessible in the short variant but hidden into the HLTFMet1 conformation, at least in non-denaturing conditions. In addition both peptides belong to the HLTF DNA binding domain that is highly conserved in the available mammalian sequences (GenBank accession numbers: mouse: #AF165911, rabbit: #U66564 and human: #AJ418064).

The specificity of these antisera was checked by immunoprecipitation of the human HLTF variants expressed by transcription/translation in a rabbit reticulocyte lysate in the presence of [³⁵S] cysteine. The HLTF cDNA template (pGEM-4Z-HLTF; (Ding,H. et al. (1996) DNA Cell Biol., 15, 429-442)) drove synthesis of the two protein variants with apparent molecular weights of 110 (HLTFMet123) and 130 kDa (HLTFMet1) on SDS-PAGE, together with incomplete translation products (Fig.1, lanes 2 and 4). As expected, the ART2 antiserum reacted specifically with HLTFMet1 (lane 3).The ASE2 antiserum immunoprecipitated HLTFMet123 together with a small amount of the larger variant (lane 1): this weak contamination should not interfere with further use of this serum. Neither antiserum reacted with the negative control, i.e *.in vitro* translated luciferase (lanes 5-8).

### Sequence of HLTF in the hamster

In order to check whether the human peptides used to raise the antisera described above were conserved in the hamster HLTF sequence, it was surmised that the 5' part of the HLTF cDNA encoding the DNA binding domain was also well conserved in the hamster, and primers were designed for reverse transcription and amplification by PCR in this region based on an alignment of the mouse, rabbit and human cDNA sequences with the Matcher software (Huang,X. & Miller,W. (1992) Adv.Appl.Math., 12, 373-381) from the EMBOSS software package. RT-PCR was performed on polyA(+) RNA extracted from HKT-1097 cells, the product was cloned into the *pCR4* vector, and its sequence determined. Surprisingly, a 100% identity with human HLTF was found over the 550 bp fragment, suggesting a sample contamination. The experiment was repeated on RNA extracted from hamster liver or testis, confirming the sequence. Two other approaches were used to demonstrate that no human cDNA contamination had occurred. The sequence of a genomic fragment amplified by PCR within exon 2 was first determined and it was again 100% identical to human, confirming the deduced peptide sequence; a similar experiment could not be performed for exon 4 because of its short size. cDNA's extending upstream from the second peptide coding region were then cloned by a 5' RACE approach: one product was found with 100% identity to the human cDNA but presenting a 22-bp segment that was clearly different. The two antisera developed against peptides of human HLTF could be used in the hamster cancer model.

### Morphological observations

It has been shown that the S100 protein was an early and specific marker of cell transformation related to DES tumourigenicity in the kidney, appearing as soon as 4 months after beginning of drug exposure (Toubeau,G. et al. (2001) Histochem.Cell Biol., 115, 429-438). Thus, kidneys of animals terminated at different time points during DES treatment were processed for S100 as well as HLTF immunostaining. No morphological evidence of HLTF expression was noted in the renal cortex of control animals, but a few collecting tubules were found HLTF positive in the inner medulla of some individuals. This point was not pursued further. Kidneys of hamsters treated with DES for one month still appeared negative for both HLTF and S100. A first evidence of immunoreactive HLTF associated with early signs of neoplastic transformation was seen in the renal tissue of an animal sacrificed two months after beginning of exposure (Fig.2, A). Kidneys of this individual and of the other animals of the same group were still S100 negative. HLTF positive cells were a consistent finding in medium size and large tumors of animals treated with DES for 6 months or more. HLTF expression either occurred in scattered cells (Fig.2, B-C), or was seen in tumor cell groups (Fig.2, D). Of note, with regard to immunostaining intensity and distribution, no difference was observed between the ASE2 and ART2 antisera. The appearance of S100 immunoreactivity was posterior to that of HLTF since S100 positive cells were only seen after four months of DES exposure. Later on, S100 and HLTF immunoreactivities were not systematically associated. Tumor cell populations could be HLTF positive and S100 negative (Fig.3, A-B), HLTF negative and S100 positive (Fig.3, C-D) or could exhibit both markers (Fig.3, E-F). In the latter case, however, no clear-cut evidence of colocalization was found (i.e. HLTF and S100 immunoreactivities occurred in distinct cells).

As shown previously, the HKT-1097 cell line derived from DES-induced renal tumors shares a variety of markers with the neoplasms of origin (Brohee,R et al. (2000) In Vitro Cell Dev.Biol.Anim, 36, 640-649; Laurent,G et al. (1999) In Vitro Cell Dev.Biol.Anim, 35, 339-345). Immunofluorescence staining clearly demonstrated HLTF expression in HKT-1097 cells (Fig.4). Simultaneous detection of vimentin (an intermediate filament constitutively expressed by HKT-1097 cells) and HLTF by double label immunofluorescence revealed an equivalent level of HLTF expression (as inferred from signal intensity) in all cells (Fig.4, A-B, E-F). Besides, combined detection of lamin B (an intermediate filament associated with the nuclear envelope) and HLTF unequivocally localized the latter protein in cell nuclei (Fig.4, C-D, G-H). Similar immunofluorescence patterns were found with the ASE2 (Fig.4, B,D) and ART2 antisera (Fig.4, G,F), although the signal periphery appeared less sharp with the latter, suggesting a more heterogeneous distribution of the HLTFMet1 protein.

The tumorigenic potential of the HKT-1097 cell line has been demonstrated by its ability to give rise to tumor xenografts in nude mice. As illustrated in Figure 3 (E, F), HLTF immunostaining in HKT-1097-derived neoplasm revealed the presence of scattered positive cells throughout the tumor tissue. This suggests that HLTF expression persists for many generations in a subpopulation of tumor cells.

### Obtention and characterization of hybridoma

### Monoclonal antibodies directed against HLTFMet123

### MAb validation

HLTF8F1 hybridoma was selected because of its ability to produce monoclonal antibodies specifically directed against HLTFMet123. Those antibodies are IgG1. These were characterized by ELISA against the recombinant 123-270 amino acid domain of HLTFMet123. In addition, the MAbs were assayed by immunoprecipitation of the human HLTF variants expressed by transcription/translation in a rabbit reticulocyte lysate in the presence of [³⁵S] cysteine. They only immunoprecipitate the HLTFMet123 variant. A deposit has been made according to the Budapest Treaty for this hybridoma under the deposit number LMBP 6387CB on May 3, 2005 at the Belgian Coordinated Collections of Microorganisms (BCCM), Laboratorium voor Moleculaire Biologie - Plasmidencollectie (LMBP), Universiteit Gent, Technologiepark 927 (FSVM-bldg), B-9052 Gent-Zwynaarde, Belgium.

### Sequence determination of HLTF8F1 immunoglobulin light and heavy chains

As the HLTF8F1 hybridoma proliferates very slowly, the person skilled in the art may also produce the antibodies or hypervariable portion thereof by genetic engeneering. The cDNA sequence coding the variable regions of IgG1 light and heavy chains was determined by 5'RACE and RT-PCR techniques. The sequences obtained are presented in tables 1 and 2 and in figures 5 and 6.

**Table 1: The germline gene sequences with the highest identity with the sequence of the HLTF8F1 different regions are given below. The sequences are presented by their GenBank accession numbers.**

| | Light chain | | | | Heavy chain | | | |
|---|---|---|---|---|---|---|---|---|
| | Leader sequence | V region | J region | C region | Leader sequence | V region | J region | C region |
| HLTF8F1 | M20830.1 | κV1-135 | κJ1 | κC | AAC53258.1 | V14S3 | J4 | CG1 |

The IGHD and IGKD regions cannot be assigned to a germline counterpart since deletions probably occurred during the D-J and the V-DJ recombinations. The IGHD gene seems to have almost disappeared during the recombinations since only 7 nucleotides link the putative IGHV and IGHJ region. The IGKD is completely absent.

**Table 2: Comparison between the light and heavy chain sequences of HLTF8F1 and their corresponding germlines. The mutations were counted and located in the structure of the MAb.**

| | Light chain | | | | | | Heavy chain | | | | | |
|---|---|---|---|---|---|---|---|---|---|---|---|---|
| | FR1 | CDR1 | FR2 | CDR2 | FR3 | CDR3 | FR1 | CDR1 | FR2 | CDR2 | FR3 | CDR3 |
| Nucleotides | 0 | 1 | 0 | 0 | 0 | 1 | 1 | 0 | 1 | 0 | 4 | 5 |
| Amino acids | 0 | 1 | 0 | 0 | 0 | 2 | 1 | 0 | 0 | 0 | 3 | 2 |

FR segments (framework) correspond to the low variability segment present in the variable region and CDR segments to the high variability segments.

### Monoclonal antibodies directed against HLTFMet1

Seven hybridomas producing monoclonal antibodies specifically directed against HLTFMet1 were developed. They were validated by ELISA against the recombinant 1-208 amino acid domain of HLTFMet1 and by immunoprecipitation of the human HLTF variants expressed by transcription/translation in a rabbit reticulocyte lysate in the presence of [³⁵S] cysteine. Two of them were positive for our screening conditions: HLTF6-1 and HLTF11-1. Both hybridoma produce IgG1.

### Monoclonal antibodies directed against HLTFMet1 and HLTFMet123

Two monoclonal antibodies are specifically directed against both HLTF variants: HLTF5A8 and HLTF5-1. They were validated by ELISA against the recombinant 1-208 amino acid domain of HLTFMet1 and against the recombinant 123-270 amino acid domain of HLTFMet123. In addition, they were assayed by immunoprecipitation of the human HLTF variants expressed by transcription/translation in a rabbit reticulocyte lysate in the presence of [³⁵S] cysteine. In ELISA, only HLTF5-1 is immunoreactive against HLTFMet1 whereas both MAb immunoprecipitate HLTFMet1 and HLTFMet123. This observation may be the result of different epitopes recognized by those MAbs, epitopes which may differently be exposed after HLTFMet1 coating on ELISA plate. Of course, this problem is not present in immunoprecipitation of the complete protein.

A deposit has been made according to the Budapest Treaty for HLTF5A8 hybridoma under the deposit number LMBP 6388CB on February 15, 2005 at the Belgian Coordinated Collections of Microorganisms (BCCM), Laboratorium voor Moleculaire Biologie - Plasmidencollectie (LMBP), Universiteit Gent, Technologiepark 927 (FSVM-bldg), B-9052 Gent-Zwynaarde, Belgium.

To summarize, the results obtained in the present invention show that HLTF protein expression was detected at early stages of development of renal tumours induced by DES in Syrian golden hamsters. The protein was even detected before the appearance of S100, an early tumour marker previously identified in this model: indeed two months after initiating DES administration, tumour buds consisting of only a few dozen cells that all exhibited strong immunostaining with the antisera against HLTF were already found.

Genetic studies in human and mouse have shown that inactivating mutations or deletions of genes encoding subunits of the SWI/SNF complex were associated with cancer, qualifying these genes as putative tumour suppressors. In keeping with this idea, inhibition of *HLTF* gene expression caused by promoter hypermethylation was recently observed in 30-70% of human colon cancer and human gastric carcinoma (Hamai,Y. et al. (2003) Cancer Sci., 94, 692-698). The data presented here on an animal model of kidney carcinogenesis are in sharp contrast with previously published observations, and suggest instead that the *HLTF* gene behaves like an oncogene. In fact, in the very study where *HLTF* was initially found to be inactivated in a number of colon and gastric cancers, neither *HLTF* promoter hypermethylation nor loss of expression was found in cancers of other organs such as the breast and the lung (Moinova,H.R. et al. (2002) Proc.Natl.Acad.Sci.U.S.A, 99, 4562-4567). Moreover a 20-fold increase in HLTF mRNA was observed in transformed cell lines derived from various tissues such as breast, ovary, cervix Gong,X. et al. (1997) Dev.Biol., 183, 166-182). These data were confirmed by the Applicants at the protein level by showing high levels of HLTFMet1 and HLTFMet123 in HeLa (cervix adenocarcinoma) and TE671 (rhabdomyosarcoma) cells. Intriguingly, *HLTF* over-expression was never detected in differential gene expression profiles performed between tumour tissue and its normal counterpart. An explanation might be found in the HLTF expression pattern that the applicants observed here during kidney tumour progression: although every single cell stained for HLTF in tumour buds, the HLTF signal was only detected in about 10% of cells scattered in larger tumours. If a similar pattern occurred in tumors derived from other tissues, the 10-20 fold increase in HLTF expression in 10% of tumour cells would be diluted out in the population of HLTF negative cells, and it might not be detected upon analyses of whole tumour homogenates.

Several reasons could be put forward to explain the decrease in number and the dispersion of HLTF positive cells during tumour progression. First, the few cells that form the tumoral bud might evolve to form different clonal populations showing different phenotypic drifts. Indeed, the Applicants have shown that progression of DES-induced renal tumour from tumour buds to advanced malignancies was associated with phenotypic variation and clonal divergence of neoplastic cells ( Nonclercq,D. et al. (2002) Histochem.J., 34, 487-497). Another hypothesis is that HLTF over-expression might be involved in early steps of cellular transformation, and could later be replaced by a more efficient survival strategy consisting of genetic alterations that would confer higher proliferative capacity. Indeed, HLTF expression did not seem linked to proliferation since HLTF-positive cells and proliferating cells (BrdU staining, not shown) belonged to distinct subpopulation. Other studies have indicated that HLTF expression was coupled to terminal differentiation in mouse development (Gong,X. et al. (1997) Dev.Biol., 183, 166-182): one could imagine that even if HLTF over-expression favored immortalization, it might still slow down proliferation, and that, with tumour progression, the cells might bypass this problem by *HLTF* gene hypermethylation as observed in advanced colon adenomas (Moinova,H.R. et al. (2002) Proc.Natl.Acad.Sci.U.S.A, 99, 4562-4567). The observation that some HLTF-positive cells always remain present during progression and are found scattered in the tumour mass is suggestive of a paracrine interaction: HLTF-positive cells might stimulate proliferation of the surrounding HLTF-negative cells by secretion of different growth factors.

One of the rationale behind the present study was that the *HLTF* gene encoded two protein variants with different biological functions, only the shorter of which (HLTFMet123) presented a transcriptional activity (Ding,H. et al. (1996) DNA Cell Biol., 15, 429-442; Ding,H. et al. (1999) J.Biol.Chem., 274, 19573-19580). No difference could be found in the immunostaining patterns demonstrated by the specific antisera (ART2 and ASE2) developed against the individual variants. The immunogenic peptide "specific" for HLTFMet123 is also present in HLTFMet1, and the specificity of the ASE2 serum only relies on the three-dimensional structure of the larger amino-terminal domain in HLTFMet1 where the sequence of the peptide used to raise ASE2 is buried. One could argue that the microwave pre-treatment of renal sections performed for antigen retrieval could have denatured the HLTF proteins and expose this immunogenic peptide, allowing recognition of the HLTFMet1 protein by the antiserum "specific" of HLTFMet123. However, the immunofluorescence staining of HKT-1097 cells did not involve such harsh treatment and anyway revealed very similar staining patterns for both antisera, with just a fuzzier HLTFMet1 staining at the nuclear periphery as compared to HLTFMet123 (Figure 4, B,D vs Figure 4, F,H). Interestingly the HLTF RING domain has been found to interact with an atypical Type IV P-type ATPase, a putative phospholipid pump located at the inner nuclear membrane (Mansharamani,M. et al. (2001) J.Biol.Chem., 276, 3641-3649) .

The HLTF proteins were previously shown to locate in the nucleoplasm of HeLa cells, as expected for SWI/SNF proteins (Ding,H. et al. (1996) DNA Cell Biol., 15, 429-442). In the present study, a nuclear localisation was also clearly observed in HKT-1097 cells fixed in paraformaldehyde. In contrast, in the kidney tumours fixed in alcoholic Bouin's mixture most HLTF-positive cells presented a strong cytoplasmic signal, while only few cells displayed a nuclear staining. Again, the different locations observed between cells in culture and tumoral tissues might be artefactual and caused by tissue processing (fixation, dehydratation, dewaxing) and/or the treatment applied for antigen retrieval. Alcoholic Bouin's mixture was not used for HKT-1097 cells, but experiments using another alcoholic fixation method (Carnoy) on these cells indeed showed a significant alteration of the nuclear signal. However, in a few tumour samples that were fixed in paraformaldehyde we could detect the same proportion of HLTF-positive cells with strong cytosolic staining as found in the present study, suggesting other explanations than processing artefacts. A similar conclusion was drawn in previous study on the TP53 oncoprotein (Bassarova, A.V. & Popov,A.A. (1998) Folia Histochem.Cytobiol., 36, 127-132). It showed that the cytoplasmic accumulation of TP53 was a fact and not the result of pre-treatments like poor fixation or antigen unmasking and that these actually improved the quality of the immunochemical reaction (Bassarova,A.V. & Popov,A.A. (1998) Folia Histochem.Cytobiol., 36, 127-132). Several examples are known of transcription factors or associated cofactors that shuttle between the nucleus and the cytoplasm according to various stimuli. Sequestration in a cellular compartment is indeed a way to regulate transcription activity. NFκB is a classical example: it is kept inactive as a cytoplasmic complex with IκB until the latter undergoes phosphorylation and proteosomal degradation, releasing NF_{K}B that migrates to the nucleus for transcription activation (reviewed by Rayet,B. & Gelinas,C. (1999) Oncogene, 18, 6938-6947). Conversely, MEF2 is sequestered in an inactive nuclear complex with a histone deacetylase that is translocated to the cytoplasm upon phosphorylation by a calcium-dependent kinase, allowing MEF2 to activate transcription of genes involved in terminal muscle differentiation (McKinsey,T.A. et al. (2000) Nature, 408, 106-111). One could hypothesize that sequestration of HLTF outside of the nucleus might be part of the transformed phenotype as shown previously for p27Kip, a negative regulator of the cell cycle that accumulates in the cytoplasm of colorectal tumours (Sgambato, A et al. (1999) Mol.Carcinog., 26, 172-179).

In conclusion, the data presented here show that HLTF protein expression is linked to early transformation of cancer and that HLTF protein detection could constitute a very early marker in some human cancers.

## Claims

1. A HLTF protein domain starting from amino acids 1 up to amino acids 270 or a portion thereof.

2. The portion of the HLTF protein domain according to the claim 1 which starts from amino acids 1 up to amino acids 208 or starts from amino acids 123 up to amino acids 270.

3. An inhibitor which is specifically directed against the HLTF protein domain according to claim 1 or its portion according to claim 2.

4. The inhibitor according to claim 3 which is a (monoclonal) antibody (possibly labelled) or a hypervariable portion thereof (possibly labelled).

5. The hypervariable portion of a monoclonal antibody according to claim 4 which comprises the sequence SEQ.ID.N°7 and/or SEQ.ID.N°8.

6. The inhibitor according to claim 3 which is a peptide selected from phage display technology.

7. The inhibitor according to claim 3 which is an antiserum made of the antibodies according to claim 4.

8. An hybridoma cell or a recombinant cell producing the monoclonal antibodies according to claim 4 or hypervariable portions thereof, according to claim 5.

9. The cell according to claim 8 having the deposit n° LMBP 6387CB.

10. The cell according to claim 8 having the deposit n° LMBP 6388CB.

11. A pharmaceutical composition comprising an adequate pharmaceutical carrier and the inhibitor according to any of the preceding claims 3 to 7.

12. Use of the pharmaceutical composition according claim 11 for the manufacture of a medicament in the treatment and/or the prevention of a neoplasm (tumor) in a mammal subject, including a human patient.

13. Use according to claim 12, wherein the neoplasm is a kidney tumour, preferably an early kidney tumour.

14. Use according to claim 12 wherein the neoplasm is a brain tumour.

15. Use according to claim 12, wherein the neoplasm is an ovary tumour.

16. Use according to claim 12, wherein the neoplasm is a prostate tumour.

17. A diagnostic kit for detecting the presence of an (early stage) tumour in a mammal subject, comprising the inhibitor according to any of the preceding claims 2 to 7.

18. A method for detecting (preferably, at an early stage) the presence of a tumour in a mammal subject sample and comprising the steps of :
- contacting the biological sample extracted from a mammal with the inhibitor according to any of the preceding claims 2 to 7, so as to be able to form a complex between the inhibitor and the HLTF protein, possibly present in a biological sample and,
- measuring a level of HLTF protein in the sample of the mammal there through, wherein an increase in the level of HLTF protein in the sample compared to a normal control sample indicates the presence of an (early stage) tumour in the biological sample.

19. The method according to claim 18, wherein the sample is a kidney tissue sample.

20. The method according to claim 18, wherein the sample is a brain tissue sample.

21. The method according to claim 18, wherein the sample is a tissue sample.

22. The method according to claim 18, wherein the sample is a prostate sample.
